# EUROPEAN PATENT APPLICATION

(11) **EP 1 241 247 A1**
(43) Date of publication of application: **18.09.2002**
(21) Application number: 01870044.3
(22) Date of filing: 13.03.2001
(51) Int. Cl.: C12N 1/20, C12R 1/125, C12R 1/40, C12R 1/425, A01N 63/00

(54) **Isolated bacteria for the protection of plants against phytopathogenic fungi and bacteria**

(71) Applicant: C.C.H. S.A., 1931 Zaventem (BE)
(72) Inventor:
(74) Representative: Van Reet, Joseph

(57) **Abstract**

The invention relates to antagonistic bacteria for the protection of plants against phytopathogenic bacteria and fungi. The isolated bacteria are of the species *Paenibacillus polymyxa, Pseudomonas chlororaphis, Pseudomonas putida, Serratia plymuthica* and *Bacillus subtilis.* They can be applied as a mixture or separately to control for example the following plant diseases: crown gall disease caused *Agrobacterium tumefaciens* in grape, diseases caused by *Corynebacterium spp., Pseudomonas syringae* and *Xanthomonas campestris* in tomato and cabbage, diseases caused by *Botrytis cinerea* in cucumber and tomato, powdery mildew caused by *Erysiphales spp.* in cucumber and tomato, damping-off diseases caused by *Fusarium oxysporum* in cucumber, melon and tomato, diseases caused by *Helminthosporum sativum* in cereals, the root-rot disease caused by *Rhyzoctonia solani* in cotton and beans, diseases caused by *Sclerotium rolfsii* in beans, *Dactylium dendroides* in mushrooms and others.

## Description

The present invention relates to isolated bacteria capable of inhibiting the growth of phytopathogenic fungi and bacteria.

The invention is intended for the protection of plants by the use of soil-born antagonistic micro-organisms, in particular an effective combination thereof, and/or by the use of their antagonistic metabolites. The beneficial properties of the introduced biocontrol agents should allow to suppress the development of diseases caused by pathogenic bacteria and/or fungi, to improve microbial association in the plant-soil sphere as well as to stimulate plant growth and development. The mechanisms of action of these microbial agents in the biocontrol of diseases caused by bacteria and/or fungi include competition for life space on a surface of the plant, production of pathogen suppressing metabolites, pathogen cell-wall destroying enzymes, and plant growth and immunity promoting substances.

Modern extensive agriculture would not exist without monoculture of profitable crops and without the use artificial fertilizers and chemical pesticides. Such practice leads to degenerative changes in soil microorganism community, the accumulation of plant pathogens and, as a result, to a decline of soil fertility and crop yields. Additionally, development of resistance to the fungicides can lead to the appearance of new pathogen types. This increases further the need for chemicals, the environment pollution and the thread to human health and life (Schwinn et al., 1991).

The problem of reducing the application of chemical pesticides, especially for the production of food stuff, - vegetables, fruits and other fresh-consumed plants is of top priority. The usage of natural soil inhabitant beneficial antagonistic bacteria for the biocontrol of pathogen can replace partially or fully the application of chemicals (Rodgers, 1993).

Natural soil microorganism community is stabilized due to a balance between the groups of microbes producing different antibiotic compounds. Many microorganisms beneficial for plants excrete antibiotics and therefore dominate in soil (actinomycetes, bacillus and pseudomonas). That is why beneficial for plants microbes prevail in native soil, while pathogens are normally suppressed. This phenomenon would be reasonably used to create new environmentally friendly bio-pesticides based on antagonistic microorganisms. Several active mechanisms are known to be responsible for antagonistic properties of microorganisms, they are: production of antibiotics, siderophores, cell wall digesting enzymes (chitinases and proteases). Plant and soil protective colonizing ability is highly important because microbes fight for space, food, micro-elements and change environment producing metabolites (for instance by changing pH to unfavorable values for others).

In general, a number of micro-organisms are known to produce antagonistic substances, however, practical bio-control activities in laboratories, greenhouses or limited field conditions are announced only for a few species of antagonistic bacteria, namely for *Agrobacterium, Bacillus, Enterobacter, Pseudomonas, Serratia*, and for the fungi *Trichoderma, Ampelomyces, Fusarium* (Strange R.N., 1993).

There are several examples of real success of plant diseases cure by using of artificially introduced natural antagonistic bacteria. For effective suppression of take-all and root-rot of wheat and black root-rot of tobacco caused by some phytopathogenic fungi, strains of *Pseudomonas fluorescent* producing as antibiotic substances phenazine and 2,4 diacetylphloroglucinol have been used (Weller D.M., Thomashow L.S., 1993). Production of the antibiotic compound identified as pyrrolnitrin appeared to be responsible for the ability of some *Pseudomonas cepacia* strains to control blue and grey mould of apples and pears caused by *Penicillium expansum* and *Botrytis cinerea* (Janisiewicz W.J., 1988). The *Bacillus subtilis* and *Pseudomonas syringae* strains, which are producing not yet completely identified antibiotic compounds, were used as bio-control agents for brown rot of stone fruits and Dutch elm disease (Strange R.N., 1993). Other strains of soil bacterium *Bacillus subtilis* were successfully applied for suppression of the following illnesses: stem canker and black scurf of potatoes caused by *Rhizoctonia solani*, common potato scab caused by *Streptomyces scabies, Fusarium* wilt development in chickpea (Hervas A., et al., 1998, Schmiedeknecht G., et al., 1998). In field experiments strains of *Bacillus polymyxa* were shown to be able to suppress a number of phytopathogenic fungi (Kado C.J. et al., 1987; Strange R.N., 1993; Dijksterhuis J. et al., 1999). Two isolates of *Serratia liquefaciens* were able to suppress sporulation of *Botrytis cinerea* on grape tissue and have shown effective suppression of plant disease (Whiteman S.A., Stewart A., 1998). Several antagonistic strains which belong to the *Enterobacter* species are in use as fungal plant disease protecting agents against *Phytophthora capsici* and *Pythium ultimum* (Yoon S.H., 1999; Burkhead K.D. et al., 1998). Strains of *Pseudomonas spp.* and *B. polymyxa* were able to suppress bacterial and fungal phytopathogen development on tomato in model systems of isolated leaves (Avdienko I.D. et al., 1996).

The perfect antagonist or antagonist combination must possess all above-descibed antagonistic mechanisms, but in fact manifestation of antagonistic properties of artificially introduced natural micro-organisms depends on many factors and is not well controlled. The use of combination of microorganisms which can complete each other in ability to inhibit different pathogens at different conditions will generate more predictable biocontrol management. There is an example of the application of a number of the bacterial species *Pseudomonas fluorescens, Serratia liquefaciens, Serratia plymuthica, Bacillus subtilis, Bacillus pumilis* and *Bacillus polymyxa* for effectively inhibiting the development of post-harvest diseases caused by the fungi *Botrytis cinerea* and *Alternaria brassicicola* (Leifert C. et al., 1999).

The present invention relates to novel bacterial isolates which are capable of inhibiting the growth of phytopathogenic fungi and bacteria and to combinations of these isolates which are even more effective for inhibiting the growth of particular phytopathogenic fungi and bacteria, i.e. for the prophilactic and/or curative treatment of plant diseases in different stages of plant development, compared to the individual isolates.

The isolated bacteria according to the invention comprise bacteria of at least one bacterial strain having the identifying characteristics of an isolate selected from the group consisting of *Paenibacillus polymyxa* VKPM B-7961, *Pseudomonas chlororaphis* VKPM B-8060, *Pseudomonas putida* VKPM B-7958, *Serratia plymuthica* VKPM B-7957 and *Bacillus subtilis* VKPM B-7960 and in particular bacteria of at least one strain formed by these isolates.

Samples of the novel bacterial isolates of the present invention have been deposited under the terms of the Budapest Treaty on the International Recognition of the Deposit of Microorganisms for the purpose of patent procedures at the Russian National Collection of Industrial Microorganisms (VKPM) at May 30, 2000 for the *Paenibacillus polymyxa*, the *Pseudomonas putida*, the *Serratia plymuthica*, and the *Bacillus subtilis* strains and at December 20, 2000 for the *Pseudomonas chlororaphis* strain, more particularly under the accession numbers indicated hereabove. The deposits were done in the name of Ryabchenko N.F., Mojaiskoe sh., 113,18, 143000, Odinzovo.

The scope of the present invention is not limited to the novel bacterial strains deposited with the VKPM but other strains showing the same characteristics, in particular the same antagonistic properties, obtained for example by isolating another but similar bacterial strain from nature or by genetically modifying the deposited strains are also covered by the present invention.

According to the invention it has been found that the novel bacterial isolates were particularly useful, either alone but especially in combination, for suppressing or inhibiting the growth of different phytopathogenic fungi and bacteria. The different isolates have been found to be effective to inhibit the growth of a number of different phytopahogenic bacteria and fungi on a solid nutrient medium as illustrated in Table 3. Tests have demonstrated the occurrence of synergetic effects when using combinations of a number of the different bacterial strains and this either simultaneously or separately.

The isolated bacteria according to the invention, which comprise bacteria of one to five, preferably of two or three to five, different bacterial strains each having the identifying characteristics of a different isolate selected from the above defined group, may be combined with an agriculturally compatible carrier to achieve a formulation suitable for the protection of plants against phytopathogenic bacteria and/or fungi.

The carrier may for example be water to which nutrient salts, such as for example K₂HPO₄, NaH₂PO₄, NH₄Cl, and MgSO₄, and nutrients such as sucrose (or glucose) and peptone have been added to provide a growth medium for the bacteria. The liquid formulation may also be applied onto solid carrier particles and/or dried to provide a solid formulation.

The present invention also relates to a method for the protection of plants against phytopathogenic bacteria and/or fungi, wherein the plants are treated with isolated bacteria according to the invention, in particular with a formulation according to the invention.

In a preferred embodiment of the method according to the invention, the plants are treated either simultaneously or separately (with or without a period of time between the different applications) with isolated bacteria of two or more different bacterial strains each having the identifying characteristics of a different isolate selected from the above defined group of isolates. Synergistic effects have been observed when using such combinations of bacteria.

The number of micro-organisms to be used at once (from one to five, either in one formulation or applied separately) depends on the type and development of the disease. Moreover, for each disease, the best combination of micro-organisms is preferably selected. The used bacteria possess biocontrol activity via the combined production of antibiotics, siderophores and cell-wall lytic enzymes suppressing phytopathogen growth and development, and plant growth promoting substances stimulating the immunity response of plants. At the same time the beneficial bacteria compete with pathogens for life space on the surface of the plant leaves and the roots.

The method according to the invention enables to control the following plant diseases: crown gall disease caused by the phytopathogenic bacterium *Agrobacterium tumefaciens* in grape, diseases caused by the phytopathogenic bacteria *Corynebacterium spp., Pseudomonas syringae* and *Xanthomonas campestris* in tomato and cabbage, diseases caused by fungi *Botrytis cinerea* in cucumber and tomato, powdery mildew caused by fungi *Erysiphales spp.* in cucumber and tomato, damping-off diseases caused by fungi *Fusarium oxysporum* in cucumber, melon and tomato, diseases caused by *Helminthosporum sativum* in cereals, the root-rot disease caused by fungi *Rhyzoctonia solani* in cotton and beans, diseases caused by *Sclerotium rolfsii* in beans, *Dactylium dendroides* in mushrooms and others.

The manufacturing process, which is used for the production of the biopesticide, includes the production of active substances, i.e. of the bacteria via a fermentation process and the production of the active substance, i.e. of the formulation by mixing from one to five separately growing cultures in equal proportion (for example 1:1:1:1:1) or in unequal portions. Although another agriculturally compatible carrier can be added, the medium used for the culture of the different bacteria can serve as carrier to produce the formulation, possibly in combination with another carrier.

The bacterial cultures can be obtained by fermentation in flasks or fermenters in a simple mineral medium, containing proper amounts of the following mineral components: K₂HPO₄, NaH₂PO₄, NH₄Cl, MgSO₄, sucrose (or glucose) and peptone and is composed of living cells (up to 2. 10⁹ CFU/ml).

The final compositions contain liquid suspensions (or mix on a solid carrier) of one or more of the beneficial micro-organisms: *Bacillus subtilis, Paenibacillus polymyxa, Pseudomonas chlororaphis, Pseudomonas putida* and *Serratia plymuthica* at concentrations depending on the number of bacterial suspensions which have been mixed. In case five bacterial suspensions are mixed, the formulation may contain for example up to 2.5. 10⁸ CFU/ml for each strain. The number of micro-organisms to be used at once (from one to five) depends on the type and the development of the disease.

The biopesticide can be applied in greenhouses, in field conditions and for the pre-treatment of seeds for prophylactics or disease curing at any time of plant growth. Direct plant spraying or seeds and seedling dipping is recommended. In general, strains - antagonists which are grown on simple mineral media should be diluted with water and the target plants should be sprayed with the bacterial suspension (the concentration of suspension depends on the disease heaviness). The spraying can be carried out at any plant development stage (from seeds to fruit-forming stage).

In case of seeds, they should be soaked in diluted (for example 1:1000) cultural fluid of antagonists for 1 or more hours. Then they should be placed into the soil.

For the prophylactic treatment of plants, a single treatment can be applied to seeds, plantlets or whole plants at bacterial concentrations up to 2.10⁹ CFU/l. For disease curing a regular application of the bacterial composition is recommended with bacterial cell concentrations up to 2.10¹¹ CFU/l.

This biopesticide can be applied instead of and/or together with chemical pesticides.

### Identification of the bacterial antagonists

The antagonistic bacteria have been isolated from natural soil samples and selected on broad range ability to inhibit pathogen growth in vitro as described below. For the preliminary identification of antagonistic bacteria basic microbiological methods were used: Gram's staining, observation of shape, motility (under phase contrast microscopy), physiological and biochemical tests (J.G. Holt et al., Bergey's manual of determinative bacteriology, 1994). Method of identification via 16s RNA sequencing have been applied too (Vandamme, P. et al. 1996). More details are described in Table 1 and Fig. 1, 2, 3, 4.

The bacterial strains to be used according to the invention have the following identifying characteristics:

**Table 1.**

| Description of identification properties of antagonistic Pseudomonas and Serratia strains. | | | |
|---|---|---|---|
| **Properties** | *Pseudomonas putida* | *Pseudomonas chlororaphis* | *Serratia plymuthica* |
| **Morphology:** | | | |
| Color of colony | Slightly green or yellow-green | Grey-green by time changes to the orange | Uncoloured or white |
| Diameter of colony within 2 days,28° C | 1-3 mm | 1-3 mm | 1-3 mm |
| Production fluorescent green pigment | + | + | - |
| Odor | specific | specific | specific |
| Gram stain | - | - | - |
| Motility | +, polar flagella | +, polar flagella | + |
| Cells | Direct, small sized rods, 3 x 0.8-1.0 microns | Direct, small sized rods, 3 x 0.8-1.0 microns | Direct, small sized rods, 2-4 x 0.6-0.8 microns |

| **Physiological properties:** | | | |
|---|---|---|---|
| Aerobe | + | + | Facultative anaerobe |
| catalase | + | + | + |
| oxidase | + | + | - |
| Growth limits of temperature | 5°C-38°C | 4°C-38°C | 4°C-41°C |
| Optimum of temperature | 25°C-30°C | 25°C-30°C | 25°C-35°C |
| Optimum pH | 7-8 | 7-8 | 7-8 |

| **Biochemical properties:** | | | |
|---|---|---|---|
| Type of nutrition | prototroph | prototroph | prototroph |
| Source of carbon: | | | |
| Glucose | + | + | + |
| Fructose | ND | + | ND |
| Glicerol | + | + | + |
| Sorbitol | + | - | + |
| Sucrose | ND | + | + |
| Arabinose | ND | ND | + |
| Lactose | ND | ND | + |
| Ducitol | ND | + | ND |
| Mannitol | ND | + | + |
| Xylose | ND | ND | + |
| Citrate | ND | ND | + |
| Production of acid with fermentation on glucose, glycerol, sorbitol | + | + | + |
| Production of gas with fermentation on glucose, glycerol, sorbitol | - | - | + |
| Fermentation on rhamnose | | | Production of acid is absent |
| Sources of nitrogen | Mineral salts, pepton, aminoacids | Mineral salts, pepton, aminoacids | Mineral salts, pepton, aminoacids |
| Utilization: | | | |
| Inositol | - | + | - |
| Lactose | - | - | ND |
| Maltose | ND | - | - |
| Mannitol | - | ND | ND |
| Mellobiose | - | - | ND |
| Rhamnose | - | - | ND |
| Xylose | - | - | ND |
| Arabinose | ND | - | ND |
| Galactose | ND | - | ND |
| Mellobiose | - | - | ND |
| Adonitol | - | - | - |
| Hydrolyse of gelatin | - | + | - |
| Hydrolyse of starch | - | - | - |
| Indol production | - | + | - |
| Urease production | - | ND | - |
| Argenine dihydrolase production | + | ND | - |
| Lysine decarboxylase production | - | ND | - |
| Ornithine decarboxylase production | - | ND | - |
| Hydrogen sulfide production | ND | + | - |
| Production of ammonia | ND | + | ND |
| Utilisation of: | | | |
| 1-hystidine | ND | + | ND |
| dl-lisine | ND | + | ND |
| dl-ornitine | ND | + | ND |
| 1-arginine | ND | + | ND |
| 1-proline | ND | + | ND |
| dl-serine | ND | + | ND |
| ND - not determined + - positive test result - - negative test result | | | |

**Table 2.**

| Description of identification properties of antagonistic Bacillus strains. | | |
|---|---|---|
| | Paenibacillus polymyxa | Bacillus subtilis |
| **Morphology:** | | |
| Color of colony | slightly brown | White to gray or slightly brown or rouge |
| Diameter of colony within 2 days, 35° C | 3-5 mm | 3-5 mm |
| Cells | Direct, medium sized rods, 0.6- 0.8 x 2-5 microns | Direct, medium sized rods, 2- 3 x 0.6-1.0 microns |
| Gram stain | + | + |
| Motility | + | + |
| Spores | cylindrical, central | Cylindrical, central |
| Flagella | + | + |

| **Physiological properties:** | | |
|---|---|---|
| Aerobe | Anaerobe facultative | + |
| Catalase | + | + |
| Growth limits of temperature | 5°C -45°C | 4°C -38°C |
| Optimum of temperature | 25°C-35°C | 25°C-35°C |
| Optimum pH | 7-8 | 7-8 |

| **Biochemical properties:** | | |
|---|---|---|
| Type of nutrition | prototroph | prototroph |
| Source of carbon: | | |
| Glucose | + | + |
| Arabinose | + | + |
| Lactose | + | ND |
| Mannitol | + | + |
| Glicerol | ND | + |
| Sorbitol | + | ND |
| Xylose | + | + |
| Inositol | ND | + |
| Production of acid | + | + |
| Production of gas | + | - |
| Sources of nitrogen | Mineral salts, pepton, aminoacids | Mineral salts, pepton, aminoacids |
| | | |
| Hydrolyse of gelatin | + | + |
| Hydrolyse of starch | + | + |
| Indol production | - | - |
| Levansucrase production | + | + |
| Pectinase production | + | ND |
| Growth on 10% sodium chloride | - | - |
| Growth at presence of 0.001% lysozime | - | - |
| ND - not determined + - positive test result - - negative test result | | |

A number of experiments have been performed to show the activity of the different strain against phytopathogenic fungi and bacteria in laboratory, greenhouse and field conditions. The following examples are intended to further illustrate the invention.

### EXAMPLE I: Antagonistic activity of the bacterial strains against phytopathogens in laboratory plate tests.

The antagonistic activity of the bacterial strains was examined by the two following procedures:

### In vitro assay for determination of antibacterial activity

Bacterial antagonist cells (one strain in one test) have been pricked in aseptic conditions with a loop into the middle of a Petri dish containing a solid nutrient King B medium with modification (K₂HPO₄,- 3 g, MgSO₄ · 7H₂O-0.6 g, casein hydrolisate - 8 g, sucrose or glucose - 10 g, agar - 20 g, water-up to 1l, Kado et al., 1972). The Petri dishes were incubated at 30° C for 24 hours. The bacteria were killed by chloroform steams during 40 minutes (2 drops of chloroform applied onto the inner surface of a cover part of a Petri dish). Then the dishes were kept open for 5 minutes to remove the chloroform residue followed by applying as a lawn 10ml of a semi-fluid medium containing 0.7% agar with medium described above and 0.1 ml of test-bacterial culture (concentrations 10⁸ CFU/ml). Antagonistic activity has been determined after 24 hours of incubation at 30°C by measuring the diameter of the clear zone around the place of the bacterial inoculation.

### In vitro assay for determination of antifungal activity

For this procedure potato agar plates were prepared (boil 200 g of sliced potato for 30 minutes in 1 l of water, then strain through cheesecloth. Adjust water till 1 l, add 20 g of agar, heat mixture to dissolve the agar, adjust to pH 7.0 with CaCO₃ and autoclave (Dhingra O.D., Sinclair J.B., 1987).

Fungal stock culture were grown on potato plates one week at room temperature (24° C). Conidia and mycelium were collected by sterile loop and resuspended in sterile water to a concentration 10⁸ CFU/ml (estimated by plating on potato agar plates). For preparation of fungal lawn 10 ml of liquid 0.7% agar potato medium was cooled to 40° C and inoculated with 0.1 ml of fungal stock. Then the agar was put on a surface of potato agar plates. After drying in laminar box for half an hour, bacterial antagonist cells (one strain in one test) have been pricked at the middle of the plate with fungal lawn in aseptic conditions with a loop. Plates were incubated for two days at 28° C. Antagonistic activity has been determined by measuring the diameter of the clear zone around the place of the bacterial inoculation.

Results of bacterial antagonists *Paenibacillus polymyxa* VKPM B-7961, *Pseudomonas chlororaphis* VKPM B-8060, *Pseudomonas putida* VKPM B-7958, *Serratia plymuthica* VKPM B-7957 and *Bacillus subtilis* VKPM B-7960 activity estimation are shown in Table 3.

**Table 3.**

| Activity of antagonistic bacteria against phytopathogenic bacteria and fungi bacteria in laboratory plate tests in vitro | | | | | |
|---|---|---|---|---|---|
| Phytopathogen | Antagonistic bacteria | | | | |
| | *B.subtilis* | *P.polymyxa* | *P.chlororaphis* | *P.patida* | *S.plymuthica* |
| Bacterial | | | | | |
| *Agrobacterium Tumefaciens* IDI | *+** | +++ | +++ | ++ | ++ |
| *Corynebacterium Michiganense* | *-* | *+* | +++ | *-* | - |
| *Erwinia stewari* | *-* | *+* | *-* | *-* | +++ |
| *Pseudomonas syringae pv. lachrimance* | *+* | ++ | *++* | *+* | +++ |
| *Xanthomonas campestris pv. campestris* | *+* | *+* | *-* | *+* | +++ |
| | | | | | |

| Fungal | | | | | |
|---|---|---|---|---|---|
| *Alternaria sp.* L9 | - | + | ++ | - | ++ |
| *Botrytis cinerea* SM10 | +++ | +++ | +++ | ++ | ++ |
| *Dactylium dendroides* SM21 | *++* | *+* | *-* | *-* | +++ |
| *Fusarium graminearum* L12 | +++ | *+++* | *-* | *-* | *+* |
| *Fusarium oxysporum* L15 | *++* | *++* | +++ | *+* | *+* |
| *Fusarium oxysporum f.lycopersini* | *++* | *+* | *+++* | *-* | *+* |
| *Helminthosporium sativum* L17 | *+* | *++* | *++* | *+* | *++* |
| *Mycogone perniciosa* SM5 | +++ | *++* | *+* | *-* | +++ |
| *Phomopsis sp.* SKR1 | +++ | +++ | + | - | +++ |
| *Sclerotinia sp.* SK3 | ++ | ++ | + | - | ++ |

| | | | | | |
|---|---|---|---|---|---|
| * Diameter of pathogen inhibition zones in mm: (+) - 3-10, (++) - 11-20, (+++) - 20 and more, (-) - not active. | | | | | |

Thus all tested antagonistic bacteria possess broad range activity against a number of phytopathogens. At the same time no one could act effectively against all examined phytopathogenic microorganisms. For example strain *B.subtilis* was active against 9 of the 10 fungal strains and 3 of the 5 bacterial strains. Therefore to prevent development of different types of plant diseases combination of antagonistic microorganisms would be preferable, in particular a combination of those microorganisms which show the highest efficiency against the pathogens causing the disease.

### EXAMPLE II: Sensitivity of antagonistic bacteria to fungicides

To determine sensitivity of antagonistic bacteria to a particular fungicide, the fungicide was co-inoculated with living bacterial cells (0.1 ml 10⁸ CFU/ml) in semi-liquid King B nutrient agar (0.5%) with modification (see above) and plated on Petri dishes. After that the dishes were incubated for 40 hours at 28°C. Appearance of survived bacteria lawn was taken as cells resistance to fungicide. If lawn was clear (most of the cells died) the bacterium was determined as sensitive. Concentration of fungicides was similar to the concentration used in practice. Results are shown in Table 4.

**Table 4.**

| Sensitivity of antagonistic bacteria to fungicides | | | | | |
|---|---|---|---|---|---|
| Strain | Fungicide/bacterium sensitivity | | | | |
| | Topsin-M 0.15% | NBayleton 0.2% | Benlate 0.15% | Thiram 0.2% | VITAVAX-200 0.5% |
| *Bacillus subtilis* | resistant | sensitive | sensitive | sensitive | resistant |
| *Paenibacillus polymyxa* | resistant | sensitive | sensitive | sensitive | resistant |
| *Pseudomonas chlororaphis* | resistant | resistant | resistant | sensitive | resistant |
| *Pseudomonas putida* | resistant | resistant | resistant | sensitive | resistant |
| *Serratia plymuthica* | resistant | resistant | resistant | sensitive | resistant |

All tested antagonistic bacteria are resistant to the fungicides Topsin-M and VITAVAX-200 at concentrations of 0.15 and 0.5% respectively and therefore can be used in combination with these pesticides. The strains *Pseudomonas chlororaphis, Pseudomonas putida* and *Serratia plymuthica* are further also resistant to fungicides Bayleton (0.2%) and Benlate (0.15%). All tested antagonistic bacteria were sensitive to fungicide Thiram.

### EXAMPLE III: The effects of the antagonistic bacteria in laboratory conditions on wheat seeds.

The effects of the bacterial antagonists on wheat seeds development were studied using the assay described by Grodzinski with modifications (Grodzinski A.M. et al., 1990). The seeds of winter wheat (*Triticum vulgare*) were preliminary sterilized by means of 10% hydrogen peroxide, washed with sterile water and placed overnight in sterile water. Strains of bacteria-antagonists were grown overnight in mineral medium (King B with modifications, see above) in flasks on a shaker at 30°C. For the treatment of the seeds cultural fluids with a cell concentration of 2. 10⁷ CFU /ml were applied In a first series of experiments, the seeds were treated with antagonists by dipping them in a bacterial suspension for 1 hour at room temperature (24° C). In control samples the seeds were soaked in sterile mineral medium. 10 treated full seeds were placed with their germ down on a surface of sterile wet blotter papers 5 x 15 cm in size. From the paper a roll with seeds was prepared and placed into a glass tube followed by adding 10 ml of sterile tap water. In a second series of experiments untreated seeds were placed inside paper rolls. Then 0.2 ml of antagonistic bacteria suspension were added to the rolls, followed by addition of 10 ml of sterile water. In control samples sterile mineral medium was applied. Tubes with rolls were transferred to the bioassay chamber and incubated at 24° C and a light intensity of 3000 lux/m². Three repeats were done for each sample. On the third day one drop of Knop mineral solution: Ca(NO₃)₂ x 4H₂O - 1.44 g, KNO₃ - 0.25 g, KCI - 0.12 g, KH₂PO₄ - 0.25 g, MgSO₄ x 7H₂O - 0.51g, Fe-EDTA 50 µl, microelements (mg/l): KI - 0.83, FeSO₄ - 27.8 , ZnSO₄-8.6, MnSO₄ - 22.3, CoCl₃ -0.025, CuSO₄ -0.025. was added. Water level was adjusted till original once every two days. After one week of incubation roots and first leaves length were estimated. The results are shown in Table 5.

**Table 5.**

| Effects of antagonistic bacteria on development of wheat seeds in vitro | | | | | |
|---|---|---|---|---|---|
| Experiment number | Variants | Length (cm)* | | Weight of seedlings (g)* | |
| | | Root | Leave | Raw | Dry |
| | Control | 4.5 | 7.6 | 0.15 | 0.03 |
| I | | | | | |
| | *B.subtilis* | 5.4 | 9.7 | 0.16 | 0.03 |
| | *P.polymyxa* | 4.3 | 6.0 | 0.13 | 0.03 |
| | *P.chlororaphis* | 6.4 | 9.5 | 0.17 | 0.04 |
| | *P.putida* | *4.9* | 8.2 | 0.15 | 0.03 |
| | | | | | |
| | Control | 6.3 | 9.2 | 0.25 | 0.06 |
| II | | | | | |
| | *B.subtilis* | 9.6 | 10.6 | 0.27 | 0.06 |
| | *P.polymyxa* | 4.6 | 8.4 | 0.23 | 0.06 |
| | *P.chlororaphis* | 13.0 | 13.4 | 0.28 | 0.07 |
| | *P.putida* | 7.0 | 9.4 | 0.25 | 0.06 |

| | | | | | |
|---|---|---|---|---|---|
| *Average data is presented | | | | | |

In the experiment most of the tested antagonistic bacteria were not phytotoxic to wheat seedlings. In the case of the strains *B.subtilis* and *P.chlororaphis* some stimulating effect was observed. Strain *P.polymyxa* possessed slightly suppressive effect on seedlings development.

### EXAMPLE IV: The effectiveness of the antagonistic bacteria in laboratory conditions against Fusarium infection on cucumber seeds

The antagonistic activity of *Paenibacillus polymyxa, Pseudomonas chlororaphis, Pseudomonas putida* and *Serratia plymuthica* was studied in laboratory conditions on a model of cucumber seeds treated by the phytopathogenic fungus *Fusarium oxysporum*.

The following procedure was applied: cucumber seeds (var. Parad) were preliminary sterilized by means of 1% chloramine B for 15 min, then washed in sterile distilled water. After drying the seeds were treated with pathogen and antagonists by soaking them for one hour at room temperature (24° C). Different combinations of agents and concentrations of pathogen suspension were applied. *Fusarium oxysporum* mycelium and conidia suspension were prepared as described above. In all the experiments the original fungal suspension with titre 2. 10⁸ CFU/ml was used. The strains of the bacterial-antagonists were grown overnight in a mineral medium (King B with modifications, see above) in flasks on a shaker at 30° C. For the treatment of the seeds cultural fluids with a cell concentration of 2. 10⁷ CFU /ml were applied.

Treated seeds (ten seeds in three repetitions for each sample) were placed on a surface of sterile wet blotter papers in Petri plates which were incubated at room temperature during 12 days. The plates were additionally watered every three days, to avoid drying. Symptoms of the disease development were estimated as root browning. The results are presented in Table 6.

**Table 6.**

| Activity of antagonistic bacteria against *Fusarium oxysporum* infection on cucumber seeds model | | | | | |
|---|---|---|---|---|---|
| No. | Treatment by | Pathogen dilutions | Number of seeds | Sprouts (%) | Healthy sprouts (%) |
| 1 | Control (without any treatment) | - | 30 | 80 | 100 |
| 2 | Treatment by pathogen only (*F. oxysporum*) | 1 | 30 | 93 | 21 |
| | | 1:10 | 30 | 93 | 0 |
| | | 1:100 | 30 | 87 | 8 |
| 3 | Treatment by Pathogen + *P.chlororaphis* | 1 | 30 | 93 | 7 |
| | | 1:10 | 30 | 80 | 23 |
| | | 1:100 | 30 | 87 | 42 |
| 4 | Treatment by Pathogen + *P.putida* | 1 | 30 | 80 | 17 |
| | | 1:10 | 30 | 93 | 14 |
| | | 1:100 | 30 | 80 | 100 |
| 5 | Treatment by Pathogen + *S.plymuthica* | 1 | 30 | 73 | 64 |
| | | 1:10 | 30 | 100 | 53 |
| | | 1:100 | 30 | 80 | 83 |
| 6 | Treatment by *P.chlororaphis* | - | 30 | 67 | 80 |
| 7 | Treatment by *P.putida* | - | 30 | 100 | 100 |
| 8 | Treatment by *S.plymuthica* | - | 30 | 100 | 100 |

Treatment of cucumber seeds by the phytopathogenic fungus at different concentrations has led to a strong disease development. At the same time suppression of *F.oxysporum* activity was observed in the experiments with the presence of all the antagonostic bacteria tested. Protection of seeds from fungus infection was the best in experiments with *S. plymuthica* (from 53 to 83% depending on the pathogen concentration) and *P. putida* (up to 100% at low pathogen dose). In the case of *P. chlororaphis* the results were contradictory: the percentages of healthy seeds were lower than in experiments with other antagonists, but higher than in the samples with single pathogen infection (Table 6). Seeds treatment by culture of *P. chlororaphis* can cause some inhibition of the seedling development. At the same time both the strains of *P. putida* and *S. plymuthica* can activate seeds germination compared to the untreated seeds in the control experiment. In experiments with single pathogen treatment (and in some samples with mixed treatment) the activity of the fungus was lower when higher mycelium concentration was applied (1 - without dilution comprising to 1:10 dilution). The possible explanation of this phenomenon is connected with a self-inhibition process at high fungal cell (mycelium) concentration.

### EXAMPLE V: Effect of antagonistic bacteria on development of Fusarium oxysporum f.sp.meloni on seedlings of melon.

The effect of antagonistic bacteria on the development of the phytopathogenic fungus *Fusarium oxysporum f.sp.meloni* on seedlings of melon was studied in pots (5 plants in 4 repetitions). 7 day old seedlings were artificially infected by cutting roots and soaking for 5 min in a pathogen suspension with a concentration of 10⁷ CFU/ml. After the inoculation, the seedlings were planted in sand. Seedlings soaked in water were used as a control. Antagonistic bacteria were applied at the day of inoculation and at the 8th day in the form of a suspension concentrate 1. 10⁷ CFU/ml. Bacterial and fungal suspensions were prepared as described above. Disease development was determined and estimated on 30th day. The results of the experiment are shown in Table 7. Treatment by *S. plymuthica* and *P. putida* reduced the disease development from 64.5 to 12-13%, whilst the use of *P. chlororaphis* did not suppress effectively the development of the disease.

**Table 7.**

| Effect of antagonistic bacteria on the development of the disease caused by *F.oxysporum f.sp.meloni* on melon seedlings | | |
|---|---|---|
| NN | Variants | Number of affected plants (%) |
| 1 | Control | 64.5 |
| 2 | *P.chlororaphis* | 58.6 |
| 3 | *P.putida* | 13.2 |
| 4 | *S.plymuthica* | 12.5 |

### EXAMPLE VI: Effect of antagonistic bacteria on the development of tomato plants in the presence of a Corynebacterium michiganese infection.

The phytopathogenic bacterium *Corynebacterium michiganese* can cause tomato cancer disease. The influence of antagonistic bacteria on the development of tomato plants in the presence of artificially introduced C. *michiganese* was studied. Experiments have been performed in plastic pots (3 plants for each sample in 3 repetitions). Preliminary grown 7 days old seedlings were washed with distillated water, artificially injured by cutting roots and soaked in a suspension of antagonistic bacteria or of *C. michiganese* (separately or mixed together) at a concentration of 5. 10⁷ CFU/ml for both. Subsequently the seedlings were planted in watered sterile sand. The pots were transferred to the bioassay chamber and incubated at 24° C and at a light intensity of 3000 lux/m². The plants were watered every day. Every week 10 ml Knop mineral solution ( see above) were added per plant as a mineral salts supplier in all samples. On the 30th day the height of the plants was determined. At the same time bacterial titre in plant-root zone was determined via titration on Petri dishes (King B medium) of soil suspension samples (5 g from each pot in 5 ml of water). The features of the bacterial colony morphology were used to identify the bacteria. The results of the experiment are shown in Table 8.

**Table 8.**

| Effect of antagonistic bacteria on the development of tomato plants in the presence of a *Corynebacterium michiganese* infection | | | | |
|---|---|---|---|---|
| Variants | Plant height* | | Bacterial titre ** (CFU/g soil) | |
| | Average length | | Antagonist | *C.michiganese* |
| | Stem | Leaves | | |
| | | | | |
| Control (without bacteria) | 7.54 | 2.67 | - | - |
| *B.subtilis* | 8.49 | 2.73 | 4.2. 10³ | - |
| *P.chlororaphis* | 10.13 | 2.32 | 3.1. 10² | - |
| *P.putida* | 11.78 | 3.12 | 2.0. 10³ | - |
| *S.plymuthica* | 8.57 | 2.74 | 2.2. 10⁴ | - |
| Mixture of antagonists | 10.02 | 3.63 | ND | - |
| | | | | |
| *C.michiganese* | 5.77 | 1.98 | - | 4.0. 10⁴ |
| *B.subtilis + C.michiganese* | 6.50 | 2.15 | 6.2. 10³ | - |
| *P.chlororaphis + C.michiganese* | 12.88 | 3.96 | - | - |
| *P.putida + C.michiganese* | 8.96 | 2.12 | 5.7. 10³ | - |
| *S.plymuthica* + *C.michiganese* | 10.26 | 2.36 | 1.7. 10⁴ | - |
| Mixture of antagonists + *C.michiganese* | 11.50 | 3.85 | ND | - |

| | | | | |
|---|---|---|---|---|
| * - Average data from all repeats | | | | |
| ** - Result from one typical repeat is shown ND - not determined | | | | |

In the experiments with artificial introduction of antagonistic and phytopathogenic bacteria a strong suppression of pathogen persistence in the soil in presence of antagonistic bacteria was observed. The phytopathogenic bacterium was isolated only from pots with only *C.michiganese* infection. The tomato plants in samples with only a *C.michiganese* infection showed symptoms of disease development: thinness of the stem and yellow spots on the leaves, while in other samples symptoms of disease development were not observed. Moreover all tested antagonists and their mixture were able to stimulate growth of tomato seedlings.

### EXAMPLE VII: The effectiveness of the antagonistic bacteria against artificial fungal infection in laboratory conditions

The activity of the bacterial antagonists against phytopathogenic fungi was studied in laboratory conditions on infection background. The following antagonists have been examined: *Paenibacillus polymyxa, Pseudomonas chlororaphis, Pseudomonas putida, Serratia plymuthica* and a mixture thereof.

Experiments were performed in laboratory greenhouse. Cucumber plants var. Parad were used. The antagonistic bacteria were grown overnight in simple mineral medium (King B with modification) at 28° C until a concentration of 1. 10⁹ CFU/ml was achieved. The phytopathogenic fungus *Botrytis cinerea* was grown on potato plates. Fungal mycelium has been collected and resuspended in distilled water as described above.

Before sowing the seeds were treated at once with a mixture of *Botrytis* mycelia suspension (1. 10⁸ propagules per ml) and washed antagonistic cells (1. 10⁸ CFU/ml). Single antagonists and mixture of strains (in equal proportion) have been applied. After treatment seeds were placed into plastic pots (20 cm in diameter) with watered non-sterile greenhouse soil and pots were transferred to laboratory greenhouse. For each variation, 12 plants with four-time repeats were applied. The development of the disease was estimated on the 40th day of the experiment as a development of necrosis, brown spots and other symptoms on the plants. The results are shown in Table 9.

**Table 9.**

| Protective effect of antagonistic bacteria against the development of a *Botrytis cinerea* infection on cucumber and tomato plants | |
|---|---|
| Variant | Affected plants (%) |
| Control - *Botrytis cinerea* | 17.9 |
| *P.polymyxa +Botrytis cinerea* | 0.0 |
| *P.chlororaphis + Botrytis cinerea* | 3.1 |
| *P.putida + Botrytis cinerea* | 0.0 |
| *S.plymuthica + Botrytis cinerea* | 3.6 |
| *P.chlororaphis + P.putida + Botrytis cinerea* | 12.5 |
| *P.putidu + S.plymuthica + Botrytis cinerea* | 5.6 |
| *P.putida + P.chlororaphis + P.putida + Botrytis cinerea* | 2.3 |

In all the experiments a protective effect of the antagonistic bacteria against an artificial infection of the phytopathogenic fungus *B. cinerea* was observed. All strains tested were able to suppress the development of the disease. The *P.putida* and *B.polymyxa* strains could suppress the development of the disease down to zero. However it must be noted that the development of cucumber plants (size of leaves and number of flowers) was smaller in the case of the *B.polymyxa* strain and partially in the case of the *P.putida* strain, compared to the samples treated with other antagonists.

### EXAMPLE VII: The effectiveness of the antagonistic bacteria against fungal infection on tomato in industrial greenhouse conditions

The protection of tomato plants by means of antagonistic bacteria against grey rot (pathogen *Botrytis cinerea)* was studied in industrial greenhouse conditions (Saransk region). In the used greenhouses there was a monoculture of tomato's for many years so that a rigid contagious background of pathogens was generated. The tomato plants (var. Obil'nie) were tested in the early fruiting phase. Plants with a substantially similar level of damage (beginning of disease) caused by *Botrytis cinerea* were selected and treated with the antagonistic bacteria. 15 plants were applied for each test. The antagonistic bacteria were grown overnight in a simple mineral medium (King B with modification) at 28° C until a concentration of 2. 10⁹ CFU/ml was achieved. The treatment was performed in the following way: before spraying the injured tissue of the tomato plants were carefully scraped or removed. Then overnight cultural fluids of the antagonists were diluted to different concentrations (from 10⁶ to 10⁸ CFU/ml) in tap water and used for spraying of affected plants up to a good washing of the treated grey rot spots. The fungal affection was determined on the 40th day after the treatment according to a 3 point scale: I point - the development of the disease proceeded (area of necrosis increased, sporulation of fungus increased); II point - development of disease was stopped but not fully (slow growth of damage area and fungus sporulation observed); III point - the development of the disease was suppressed completely. The results are shown in Table 10.

**Table 10.**

| Effect of the antagonistic bacteria on the development of grey rot on tomato in industrial greenhouse conditions | | | | |
|---|---|---|---|---|
| Variants | Cells titre (CFU/ml of spray solution) | Development of grey rot disease (% of tomato plants with affection according to scale) | | |
| | | I | II | III |
| Control (infected but not treated by antagonists) | - | 73 | 27 | 0 |
| Treatment with | | | | |
| *B.subtilis* | 10⁶ | 60 | 40 | 0 |
| | 10⁸ | 7 | 26 | 67 |
| *P.polymyxa* | 10⁶ | 67 | 26 | 7 |
| | 10⁸ | 13 | 33 | 54 |
| *P.putida* | 10⁸ | 80 | 13 | 7 |
| *S.plymuthica* | 10⁸ | 33 | 40 | 27 |
| Mixture of antagonists | 10⁶ | 20 | 20 | 60 |
| Mixture of antagonists | 10⁸ | 13 | 13 | 74 |

The experiment has shown that single antagonistic bacteria *B. subtilis* and *P. polymyxa* were able to suppress grey rot development at high level of cell concentration, while a mixture of the antagonists could act at low dose. Treatments with only the antagonistic bacteria *P. putida* and *S. plymuthica* at the same conditions were not effective. A Mixture of the antagonistic strains was effective at a low bacterial cell concentration (10⁶ CFU/ml) indicating the occurrence of synergetic effects.

### EXAMPLE VIII: The effectiveness of the antagonistic bacteria against powdery mildew in industrial greenhouse conditions

The biocontrol activity of the single bacterial antagonists *Bacillus subtilis, Paenibacillus polymyxa, Pseudomonas chlororaphis, Pseudomonas putida, Serratia plymuthica* and of a mixture of these antagonistic bacteria was examined in industrial greenhouse conditions.

To investigate the protective activity of the antagonistic bacteria against powdery mildew damage of tomato seeds caused by *Erysiphae* spp. on a natural background the following experiments were performed. The experiments were done in ceramic vessels 20 cm in diameter and 18 cm in depth in peat-humus soil. 12 seeds of the tomato variety "Verlioka" were sowed in each vessel. Each experimental variant included at least 3 vessels (36 plants). All experiments were done in four repetitions. Pre-sowing treatment of seeds was performed by soaking the seeds in a bacterial suspension with a density of 1. 10⁹ CFU/l during 8 hours, followed by drying at room temperature. In case a mixture of the antagonistic bacteria was used, the bacteria were present in equal proportions (1:1:1:1:1) giving a total bacterial concentration of 1 x 10⁹ CFU/l. Untreated seeds served as a control. Results were estimated every 3 days during two months. It was found that nearly all the antagonistic strains and especially the mixture thereof can stimulate the germination of the seeds (Table 11).

**Table 11:**

| Influence of the antagonistic bacteria on the germination of tomato seeds and on the affection of the seedlings by powdery mildew. | | | | | | | |
|---|---|---|---|---|---|---|---|
| Experiment | Number of seeds | Average number of seedlings per repetition in one variant after: | | | | Average number of affected plants at the end of experiment | |
| | | 10 days | | 2 months | | | |
| | | Aver. number | % | Aver. number | % | number | % |
| Control | 36 x 4 | 30.5 | 84.7 | 25.0 | 69.4 | 4.0 | 16.0 |
| *Bacillus subtilis* | 36 x 4 | 34.2 | 95 | 32.0 | 88.8 | 2.5 | 7.8 |
| *Paenibacillus polymyxa* | 36 x 4 | 36 | 100 | 31.0 | 86.1 | 1.5 | 3.2 |
| *Pseudomonas chlororaphis* | 36 x 4 | 29.7 | 82.5 | 26.0 | 72.0 | 3.2 | 12.3 |
| *Pseudomonas putida* | 36 x 4 | 35.5 | 98.6 | 33.0 | 91.6 | 3.0 | 9.0 |
| *Serratia plymuthica* | 36 x 4 | 31.1 | 86.1 | 29.0 | 80.5 | 1.2 | 4.1 |
| Mixture of strains | 36 x 4 | 36.0 | 100 | 35.5 | 98.6 | 0.5 | 1.4 |

The strains *Pseudomonas putida* and *Paenibacillus polymyxa* as well as a mixture of the different strains had the largest effect in raising the germination of the seeds. Nearly all seedlings remained alive after the treatment with the mixture of the different strains. Powdery mildew appeared after 2 months in all variants. Nevertheless only a few seedlings were affected by the disease in the variants treated with *Serratia plymuthica, Paenibacillus polymyxa* and with a mixture of the different antagonistic bacteria. The best effects were obtained with a mixture of the different antagonistic bacteria indicating the occurrence of synergetic effects.

To study the preventive activity of the antagonistic bacteria against the development of powdery mildew on mature tomato plants the next series of experiments was performed. Tomato plants (var. Fackel) were sprayed with the cultural fluid of antagonistic bacteria. Strains - antagonists were grown on a mineral medium as described hereabove. The spraying was carried out at the fruit - forming stage four times with an interval one week after the appearance of powdery mildew on the plants. One sample included 19 plants (one raw in industrial green house) in five repetitions (total number 95 plants).

The treatment was conducted by means of a portable sprayer. One variant (raw) was treated with 1.5 1 of cultural fluid. The upper and lower leaf halves were sprayed. The first spraying was carried out in the beginning of August, then 7 days later it was repeated 4 times. The final calculation was done in the first decade of September. The spreading of powdery mildew was slow and negligible throughout the experiment. 10 days after the last treatment, the plants were infected with powdery mildew for 4.2 to 9.5 % (Table 12). The weakest symptoms were recorded in the trial wherein the treatment was carried out by using cultural fluid of *B. polymyxa* (4.2 %) as well as a mixture of antagonists (5.2 %) which resulted in an effect better than the average effect of the three individual antagonists indicating therefore the occurrence of synergetic effects. The damage in the control experiments was 14.7 %.

**Table 12.**

| Results of treatment of tomato plants with cultural fluid of bacterial antagonists against powdery mildew in greenhouse (natural background) | | | | | | | |
|---|---|---|---|---|---|---|---|
| Treatment by antagonists | Total number of plants | Number of infected plants from repetition | | | | | Average number of infected plants 10 days after the last spraying |
| | | I | II | III | IV | V | |
| Control (non-treated) | 95 | 5 | 2 | 0 | 2 | 5 | 14.7 |
| *P.putida* | 95 | 0 | 0 | 2 | 4 | 3 | 9.5 |
| *S.plymuthica* | 95 | 0 | 4 | 1 | 0 | 3 | 8.4 |
| *B.polymyxa* | 95 | 1 | 1 | 0 | 2 | 0 | 4.2 |
| Mixture of antagonists | 95 | 0 | 0 | 5 | 0 | 0 | 5.2 |

### References

Avdienko I.D., Terent'ev M.A., Hrustalev A.V., Ryabchenko N.F. The use of isolated leaves of tomato as a model in phytopathological studies. 1996, Biotechnology (Russia), N11, 56-59.

Burkhead K.D , Slininger P.J., Schisler D.A. Biological Control Bacterium *Enterobacter cloacae* S11-T-07 (NRRL B-21050) Produces the antifungal Compound Phenylacetic Acid in Sabouraud Maltose Broth Culture. 1998, Soil Biol. Biochem. Vol. 30, Iss 5, pp. 665-667.

Dijksterhuis J., Sanders M., Gorris L.G.M., Smid E.J. Antibiosis plays a role in the context of direct interaction during antagonism of *Paenibacillus polymyxa* towards *Fusarium oxysporum.* 1999, J. Appl. Environm. Microbiol. Vol. 86, Iss 1, pp. 13-21.

Dhingra O.D., Sinclair J.B., Basic plant pathology methods, CRC Press, Inc., 1987.

Grodzinski A.M., Kostroma E.U., Shrol T.S., Hohlova I.G. Direct methods of soil and microorganism metabolites biotesting. 1990, In: Allelopathy and crop production, Kiev, (In Russian), Ed.: Ukranian Academy of Science, pp. 121-124.

Hervas A., Landa B., Datnoff L.E., Jimenezdiaz R.M. Effects of commercial and indigenous microorganisms on *Fusarium* wilt development in chickpea. 1998, Biol. Control., Vol. 13, Iss 3, pp. 166-176.

Holt J.G., Krieg N.R., Sneath P.H.A., Staley J.T., Williams S.T., Bergey's manual of determinative bacteriology. 1994 (ninth edition), Ed. Williams & Wilkins.

Janisiewicz W.J. Biological control of postharvest diseases of apples and pears with antagonistic mixtures. 1988, Phytopathology, 78, pp. 194-198.

Kado C.J. Schnathorst C., Arad H.R. Method of using *B*. *polymyxa* 9A to protect plants against *Verticillium dahleum* vilt. US Patent No. 4663162.

Kado, C.I., Heskett, M.G., and Langley, R.A. Studies on *Agrobacterium tumefaciens*: characterization of strains 1D135 and B6, and analysis of the bacterial chromosome, transfer RNA and ribosomes for tumor inducing ability. 1972. Physiol.Plant Pathology, 2, pp. 47- 57.

King, E.O., Ward, M.K., and Raney, D.E. Two simple media for the demonstration of pyocyanin and fluorescein. 1954. J. Lab. Clin. Med., 44, pp. 301-307.

Leifert C., Epton H.A. S. , Sigee D. C.. Biological control of fungal post harvest diseases with bacteria. US Patent No. 5869038.

Rodgers, "Potential of biopesticides in agriculture" Pestic Sci. (1993) 39:117-129.

Schwinn et al., "Control with chemicals" Advances in Plant Pathology: vol. 7: *Phytophtohora infestans*, the Cause of Late Blight of Potato, Ingram et al., eds., Academic Press, San Diego, (1991) 8:225-266.

Schmiedeknecht G., Bochow H., Junge H. Use of *Bacillus-Subtilis* as Bio-control Agent II Biological-Control of Potato Diseases.Zeitschrift fur Pflanzenkrankheiten und Pflanzenschutz. 1998 J. Plant Dis. And ProtectionVol. 105, Iss 4, pp. 376-386

Strange R.N. Plant disease control. Chapman & Hall, London 1993.

Vandamme, P., Pot, B., Gillis, M., de Vos, P., Kersters, K. and Swings, J. Polyphasic taxonomy, a consensus approach to bacterial systematics. 1996, Microbiol. Rev. 60, pp. 407-438.

Weller D.M., Thomashow L.S. Use of rhizobacteria for bio-control. Current Opinion in Biotechnol., 1993, 4, pp. 306-311

Whiteman S.A., Stewart A. Suppression of *Botrytis-Cinerea* Sporulation on Irradiated Grape Leaf Tissue by the Antagonistic Bacterium *Serratia-Liquefaciens*. 1998, New Zeland J. Of Crop and Horicultural science Vol 26, Iss 4, pp. 325-330.

Yoon S.H. Molecular Cloning and Expression of Genes Related to Antifungal Activities from *Enterobacter* Sp B54 Antagonistic to *Phytophthora capsici*, 1999, J. Microbiol. And Biotechnol., Vol. 9, Iss 3, pp. 352-357.

SEQUENCE LISTING
<110> CCH S.A.
<120> Isolated bacteria for the protection of plants against
phytopathogenic fungi and bacteria
<130> Bacterial composition
<140>
<141>
<160> 4
<170> PatentIn Ver. 2.1

<210> 1
<211> 486
<212> DNA
<213> Serratia plymuthica
<220>
<221> gene
<222> (1)..(486)
<223> 16S rRNA gene sequence of Serratia plymuthica VKPM B-7957
<400> SEQ ID NO. 1
atcatggctc agattgaacg ctggcggcag gcctaacaca tgcaagtcga gcggtagcac 60
aggvgagctt gctccctggg tgacgagcgg cggacgggtg agtaatgtct gggaaactgc 120
ctgatggagg gggataacta ctggaaacgg tagctaatac cgcataacgt ctacggacca 180
aagtggggga ccttcgggcc tcacgccatc agatgtgccc agatgggatt agctagtagg 240
tggggtaatg gctcacctag gcgacgatcc ctagctggtc tgagaggatg accagccaca 300
ctggaactga gacacggtcc agactcctac gggaggcagc agtggggaat attgcacaat 360
gggcgcaagc ctgatgcagc catgccgcgt gtgtgaagaa ggccttaggg ttgtaaagca 420
ctttcagcga ggaggaaggg tagtgtgtta atagcacatt gcattgacgt tactcgcaga 480
agaagc 486

<210> 2
<211> 489
<212> DNA
<213> Pseudomonas putida
<220>
<221> gene
<222> (1)..(489)
<223> 16S rRNA gene sequence of Pseudomonas putida VKPM B-7958

<400> SEQ ID NO. 2
gatcatggct cagattgaac gctggcggca ggcctaacac atgcaagtcg agcggatgag 60
aagagcttgc tcttcgattc agcggcggac gggtgagtaa tgcctaggaa tctgcctggt 120
agtgggggac aacgtttcga aaggaacgct aataccgcat acgtcctacg ggagaaagca 180
ggggaccttc gggccttgcg ctatcagatg agcctaggtc ggattagcta gttggtgagg 240
taatggctca ccaaggcgac gatccgtaac tggtctgaga ggatgatcag tcacactgga 300
actgagacac ggtccagact cctacgggag gcagcagtgg ggaatattgg acaatgggcg 360
aaagcctgat ccagccatgc cgcgtgtgtg aagaaggtct tcggattgta aagcacttta 420
agttgggagg aagggcagta agataatacc ttgctgtttt gacgttaccg acagaataag 480
caccggcta 489

<210> 3
<211> 488
<212> DNA
<213> Bacillus subtilis
<220>
<221> gene
<222> (1)..(488)
<223> 16S rRNA gene sequence of Bacillus subtilis VKPM B-7960
<400> SEQ ID NO. 3
tggctcagga cgaacgctgg cggcgtgcct aatacatgca agtcgagcgg acagatggga 60
gcttgctccc tgatgttagc ggcggacggg tgagtaacac gtgggtaacc tgcctgtaag 120
actgggataa ctccgggaaa ccggggctaa taccggatgs ttgtttgaac cgcatggttc 180
agacataaaa ggtggcttcg gctaccactt acagatggac ccgcggcgca ttagctagtt 240
ggtgaggtaa cggctcacca aggcaacgat gcgtagccga cctgagaggg tgatcggcca 300
cactgggact gagacacggc ccagactcct acgggaggca gcagtaggga atcttccgca 360
atggacgaaa gtctgacgga gcaacgccgc gtgagtgatg aaggttttcg gatcgtaaag 420
ctctgttgtt agggaagaac aagtgccgtt caaatagggc ggcaccttga cggtacctaa 480
ccagaaag 488

<210> 4
<211> 495
<212> DNA
<213> Paenibacillus polymyxa
<220>
<221> gene
<222> (1)..(495)
<223> 16S rRNA gene sequence of Paenebacillus polymyxa VKPM B-7961

<400> SEQ ID NO. 4
gatcatgctc aggacgaacg ctggcggcgt gcctaataca tgcaagtcga gcgggrttat 60
tagaagcttg cttctaaata ayctagcggc ggacgggtga gtaacacgta ggcaacctgc 120
ccacaagaca gggataacta ccggaaacgg tagctaatac ccgatacatc cttttcctgc 180
atgggagaag gaggaaaggc ggagcaatct gtcacttgtg gatgggcctg cggcgcatta 240
gctagttggt ggggtaawgg cctaccaagg cgacgatgcg tagccgacct gagagggtga 300
tcggccacac tgggactgag acacggccca gactcctacg ggaggcagca gtagggaatc 360
ttccgcaatg ggcgaaagcc tgacggagca acgccgcgtg agtgatgaag gttttcggat 420
cgtaaagctc tgttgccagg gaagaacgtc ttgtagagta actgctacaa gagtgacggt 480
acctgagaag aaagc 495

<210> 5
<211> 285
<212> DNA
<213> Pseudomonas chlororaphis
<220>
<221> gene
<222> (1)..(285)
<223> 16S rRNA gene sequence of Pseudomonas chlororaphis VKPM B-8060
<400> SEQ ID NO. 5
cctcacgcta ttagatgagc ctaggtcgga ttagctagtt ggtgaggtaa tggctcacca 60
aggcgacgat ccgtaactgg tctgagagga tgatcagtca cactggaact gagacacggt 120
ccagactcct acgggaggca gcagtgggga atattggaca atgggcgaaa gcctgatcca 180
gccatgccgc gtgtgtgaag aaggtcttcg gattgtaaag cactttaagt tgggaggaag 240
ggcagttacc taatacgtga ttgttttgac gttaccgaca gaata 285

## Claims

1. Isolated bacteria capable of inhibiting the growth of phytopathogenic fungi and bacteria and comprising bacteria of at least one bacterial strain having the identifying characteristics of an isolate selected from the group consisting of *Paenibacillus polymyxa* VKPM B-7961, *Pseudomonas chlororaphis* VKPM B-8060, *Pseudomonas putida* VKPM B-7958, *Serratia plymuthica* VKPM B-7957 and *Bacillus subtilis* VKPM B-7960.

2. Isolated bacteria according to claim 1, **characterised in that** said bacterial strain is selected from the group consisting of *Paenibacillus polymyxa* VKPM B-7961, *Pseudomonas chlororaphis* VKPM B-8060, *Pseudomonas putida* VKPM B-7958, *Serratia plymuthica* VKPM B-7957 and *Bacillus subtilis* VKPM B-7960.

3. Isolated bacteria according to claim 1 or 2, **characterised in that** the bacteria of said bacterial strain have the identifying characteristics of isolate *Paenibacillus polymyxa* VKPM B-7961 and are able to inhibit the growth of *Agrobacterium tumefaciens, Corynebacterium michiganese, Erwinia stewari, Pseudomonas syringae pv. lachrimance, Xanthomonas campestris pv. campestris, Alternaria sp., Botrytis cinerea, Dactylium dendroides, Fusarium graminearum, Fusarium oxysporum, Helminthosporium sativum, Mycogone perniciosa, Phomopsis sp.* and *Sclerotinia sp.*

4. Isolated bacteria according to claim 1 or 2, **characterised in that** the bacteria of said bacterial strain have the identifying characteristics of isolate *Pseudomonas chlororaphis* VKPM B-8060 and are able to inhibit the growth of *Agrobacterium tumefaciens, Corynebacterium michiganese, Pseudomonas syringae pv. lachrimance, Alternaria sp., Botrytis cinerea, Fusarium oxysporum, Helminthosporium sativum, Mycogone perniciosa, Phomopsis sp.* and *Sclerotinia sp*.

5. Isolated bacteria according to claim 1 or 2, **characterised in that** the bacteria of said bacterial strain have the identifying characteristics of isolate *Pseudomonas putida* VKPM B-7958 and are able to inhibit the growth of *Agrobacterium tumefaciens, Pseudomonas syringae pv. lachrimance, Xanthomonas campestris pv. campestris, Botrytis cinerea, Fusarium oxysporum* and *Helminthosporium sativum.*

6. Isolated bacteria according to claim 1 or 2, **characterised in that** the bacteria of said bacterial strain have the identifying characteristics of isolate *Serratia plymuthica* VKPM B-7957 and are able to inhibit the growth of *Agrobacterium tumefaciens, Erwinia stewari, Pseudomonas syringae pv. lachrimance, Xanthomonas campestris pv. campestris, Alternaria sp., Botrytis cinerea, Dactylium dendroides, Fusarium graminearum, Fusarium oxysporum, Helminthosporium sativum, Mycogone perniciosa, Phomopsis sp.* and *Sclerotinia sp.*

7. Isolated bacteria according to claim 1 or 2, **characterised in that** the bacteria of said bacterial strain have the identifying characteristics of isolate *Bacillus subtilis* VKPM B-7960 and are able to inhibit the growth of *Agrobacterium tumefaciens, Pseudomonas syringae pv. lachrimance, Xanthomonas campestris pv. campestris, Botrytis cinerea, Dactylium dendroides, Fusarium graminearum, Fusarium oxysporum, Helminthosporium sativum, Mycogone perniciosa, Phomopsis sp.* and *Sclerotinia sp.*

8. Isolated bacteria according to any one of the claims 1 to 7, **characterised in that** they comprise at least two, preferably at least three, different bacterial strains each having the identifying characteristics of a different isolate selected from the group consisting of *Paenibacillus polymyxa* VKPM B-7961, *Pseudomonas chlororaphis* VKPM B-8060, *Pseudomonas putida* VKPM B-7958, *Serratia plymuthica* VKPM B-7957 and *Bacillus subtilis* VKPM B-7960.

9. Isolated bacteria according to claim 8, **characterised in that** they comprise bacteria of a first bacterial strain having the identifying characteristics of isolate *Paenibacillus polymyxa* VKPM B-7961, bacteria of a second bacterial strain having the identifying characteristics of isolate *Pseudomonas putida* VKPM B-7958 and bacteria of a third bacterial strain having the identifying characteristics of isolate *Serratia plymuthica* VKPM B-7957.

10. Isolated bacteria according to claim 9, **characterised in that** they further comprise bacteria of a fourth bacterial strain having the identifying characteristics of isolate *Bacillus subtilis* VKPM B-7960 and preferably of a fifth bacterial strain having the identifying characteristics of isolate *Pseudomonas chlororaphis* VKPM B-8060.

11. A formulation suitable for the protection of plants against phytopathogenic bacteria and/or fungi, **characterised in that** it comprises isolated bacteria according to any one of the claims 1 to 10 and an agriculturally compatible carrier therefor.

12. A method for the protection of plants against phytopathogenic bacteria and/or fungi, **characterised in that** said plants are treated with isolated bacteria according to any one of the claims 1 to 10 and in particular with a formulation according to claim 11.

13. A method according to claim 12, **characterised in that** said plants are treated either simultaneously or separately with isolated bacteria of at least two, preferably at least three, different bacterial strains each having the identifying characteristics of a different isolate selected from the group consisting of *Paenibacillus polymyxa* VKPM B-7961, *Pseudomonas chlororaphis* VKPM B-8060, *Pseudomonas putida* VKPM B-7958, *Serratia plymuthica* VKPM B-7957 and *Bacillus subtilis* VKPM B-7960.

14. A method according to claim 13, **characterised in that** said plants are treated either simultaneously or separately with isolated bacteria of a first bacterial strain having the identifying characteristics of isolate *Paenibacillus polymyxa* VKPM B-7961, with isolated bacteria of a second bacterial strain having the identifying characteristics of isolate *Pseudomonas putida* VKPM B-7958 and with isolated bacteria of a third bacterial strain having the identifying characteristics of isolate *Serratia plymuthica* VKPM B-7957.

15. A method according to claim 14, **characterised in that** said plants are further treated either simultaneously or separately with isolated bacteria of a fourth bacterial strain having the identifying characteristics of isolate *Bacillus subtilis* VKPM B-7960 and preferably also with isolated bacteria of a fifth bacterial strain having the identifying characteristics of isolate *Pseudomonas chlororaphis* VKPM B-8060.
